# EUROPEAN PATENT APPLICATION

(11) **EP 4 223 349 A1**
(43) Date of publication of application: **09.08.2023**
(21) Application number: 20956352.7
(22) Date of filing: 02.10.2020
(51) Int. Cl.: A61M 25/00, A61M 25/09

(54) **LONG MEDICAL INSTRUMENT**

(71) Applicant: Asahi Intecc Co., Ltd., Seto-shi, Aichi 489-0071 (JP)
(72) Inventor: NOGUCHI, Naoki, Seto-shi, Aichi 489-0071 (JP); KOSUGI, Tomoki, Seto-shi, Aichi 489-0071 (JP); NAKANISHI, Yuta, Seto-shi, Aichi 489-0071 (JP); GOTO, Kenta, Seto-shi, Aichi 489-0071 (JP)
(74) Representative: Winter, Brandl - Partnerschaft mbB
(86) International application number: PCT/JP2020/037644
(87) International publication number: WO 2022/070424

(57) **Abstract**

An elongated medical instrument includes a base material part, and an outer layer part made of a resin provided on a surface of the base material part. An outer surface of the outer layer part is a substantially flat surface other than the presence of irregularly disposed recesses or protrusions.

## Description

### CROSS REFERENCE TO RELATED APPLICATION

This application is a continuation application of International Application No. PCT/JP2020/037644, filed October 02, 2020. The contents of this application are incorporated herein by reference in their entirety.

### TECHNICAL FIELD

The present disclosure relates to an elongated medical instrument.

### BACKGROUND

A technique is known in which coating the surface of a guide wire with a fluororesin, and forming a plurality of fine protrusions on the surface of the coating made of the fluororesin reduces the frictional resistance with the inner surface of a catheter or the inner wall of a blood vessel (Patent Literatures 1 and 2).

### CITATION LIST

### Patent Literature

Patent Literature 1: International Publication No. 2009/081844 Patent Literature 2: Japanese Unexamined Patent Application Publication No. 2008-125523

### SUMMARY

### TECHNICAL PROBLEM

The conventional technique has room for improvement in terms of the sliding properties.

Therefore, the present disclosure provides an elongated medical instrument having improved sliding properties.

### SOLUTION TO PROBLEM

In order to achieve such an object, an elongated medical instrument according to an aspect of the present disclosure includes: a base material part; an outer layer part made of a resin provided on a surface of the base material part; wherein a substantially flat surface of the outer layer part is provided with irregularly disposed recesses.

The substantially flat surface may be a surface corresponding to the region of the outer layer part under which the coil is not disposed.

The shape of the recesses may be that of an irregularly shaped linear portion.

The shape of the recesses may include a spatial frequency of 3 to 10 (l/mm).

The elongated medical instrument may include: a base material part; an outer layer part made of a resin provided on a surface of the base material part; wherein a substantially flat surface of the outer layer part has irregularly disposed recesses, and at least one of Conditions 1 to 9 below are satisfied when a measurement result of an outer diameter dimension along a longitudinal axis direction of the outer layer part is analyzed by FFT (Fast Fourier Transform).

(Condition 1) A value of at least either a median power frequency (referred to as MDF below) or a mean power frequency (referred to as MPF below) is 4 to 7 (l/mm). (Condition 2) At least one of the MDF or the MPF is 5 (l/mm) or more. (Condition 3) There are 10 or more spatial frequencies in which the power value is 0.01 or more. (Condition 4) The spatial frequencies with a power value of 0.01 or more are distributed from 1 to 10 (l/mm). (Condition 5) A distribution ratio in a range of an average frequency component value plus or minus 1 (l/mm) is less than 35%. (Condition 6) A distribution ratio in a range of the average frequency component value plus or minus 4 (l/mm) is approximately 70% or more. (Condition 7) A difference between the distribution ratio in the range of the average frequency component value plus or minus 1 (l/mm) and the distribution ratio of plus or minus 4 (l/mm) is approximately 40% or more. (Condition 8) A power spectrum of the spatial frequencies 3 to 10 (l/mm) is 50% or more. (Condition 9) A power spectrum of the spatial frequencies 3 to 10 (l/mm) is 70% or more.

The elongated medical instrument may include: a base material part; an outer layer part made of a resin provided on a surface of the base material part; wherein a substantially flat surface of the outer layer part has irregularly disposed recesses, and a measurement result of an outer diameter dimension along a longitudinal axis direction of the outer layer part may satisfy at least one of conditions (10) or (11) below.

(Condition 10) An outer diameter variation ratio with respect to a minimum outer diameter value is 3% or more. (Condition 11) Ten or more locations exist in a range of 3 cm in the longitudinal axis direction in which a change in an outer diameter is 5 pm or more in a range of 1 cm in the longitudinal axis direction.

In condition 10, in a case where the outer layer part includes a tapered portion, an outer diameter variation ratio excluding a change in an outer diameter due to the tapered portion may be 3% or more with respect to a minimum outer diameter value.

The outer layer part may be PFA (perfluoroalkoxyalkane), FEP (tetrafluoroethylene-hexafluoropropylene copolymer), or ETFE (tetrafluoroethylene-ethylene copolymer).

The elongated medical instrument may be a guide wire.

An outer diameter dimension of the guide wire may be 1 mm or less.

An outer diameter dimension of the guide wire may be 700 pm or less.

An average value of an outer diameter dimension of the guide wire may be 500 to 600 pm.

The elongated medical instrument may be a catheter.

### EFFECT OF THE DISCLOSED EMBODIMENTS

According to the present disclosure, the irregularly disposed recesses on the surface of the outer layer part are capable of reducing the sliding resistance with a biological wall, such as the inner wall of a blood vessel, that makes contact with the elongated medical instrument, or the sliding resistance with the inner wall of a lumen of another elongated medical instrument through which the elongated medical instrument is inserted.

### BRIEF DESCRIPTION OF DRAWINGS

FIG. 1 is a schematic diagram of a guide wire according to an embodiment of the present disclosure, and a catheter using the guide wire.
FIG. 2 is a longitudinal sectional view showing a portion of a guide wire as an example of an elongated medical instrument.
FIG. 3 is an explanatory diagram showing the process of forming recesses on the surface of a guide wire.
FIG. 4 is an external view showing a state where cracks have formed on the surface of a guide wire.
FIG. 5 is an external view showing enlarged a portion of a guide wire.
FIG. 6 is an external view showing enlarged a portion of a guide wire according to a comparative example.
FIG. 7 is a perspective view showing a device that measures the outer diameter of a guide wire.
FIG. 8 is a graph of data obtained by measuring the outer diameter dimension of a guide wire having recesses on the outer layer part along the axial direction.
FIG. 9 is a waveform diagram obtained by an FFT analysis of the measurement data in FIG. 8.
FIG. 10 is a table showing FFT analysis results of the measurement data in FIG. 8.
FIG. 11 is a table showing the results of measuring the outer diameter dimension of a guide wire having recesses on the outer layer part.
FIG. 12 is a graph of data obtained by measuring the outer diameter dimension of a guide wire according to a comparative example along the axial direction.
FIG. 13 is a waveform diagram obtained by an FFT analysis of the measurement data in FIG. 12.
FIG. 14 is a table showing FFT analysis results of the measurement data in FIG. 12.
FIG. 15 is a table of the results of measuring the outer diameter dimension of a guide wire according to a comparative example.
FIG. 16 is a table showing the results of measuring the outer diameter dimension of the tapered portion of a guide wire.
FIG. 17 is an analysis result table for another case where irregular recesses are formed on the surface of a guide wire.
FIG. 18 is a graph showing an external dimension measurement.
FIG. 19 is a graph showing FFT analysis results of the external dimension measurement data.
FIG. 20 is an analysis result table for yet another case where irregular recesses are formed on the surface of a guide wire.
FIG. 21 is a graph showing an outer diameter dimension measurement.
FIG. 22 is a graph showing FFT analysis results of the outer diameter dimension measurement data.
FIG. 23 is a graph showing a comparison of the sliding resistance between a guide wire having recesses on the surface and a guide wire having no recesses on the surface.

### DETAILED DESCRIPTION OF EMBODIMENTS

Hereinafter, an embodiment of the present disclosure will be described. The elongated medical instrument according to the present embodiment is configured as an elongated medical instrument such as a guide wire 1 or as a catheter 2. The elongated medical instrument includes a base material part 11, an outer layer part 12 made of a resin provided on a surface of the base material part 11, a substantially flat surface 121 of the outer layer part 12 is provided with irregularly disposed recesses 13.

The substantially flat surface 121 is a surface that is flat and free of recesses and protrusions, with the exception of the recesses 13 according to the present disclosure. In other words, the substantially flat surface 121 is a surface that would have no recess or protrusion if the recesses 13 according to the present disclosure were absent thereon. When an elongated medical instrument such as the guide wire 1 or the catheter 2 includes an uneven portion having a coil shape or the like, recesses and protrusions appear on a surface region corresponding to the uneven shape.

That is, the substantially flat surface 121 may be a surface region corresponding to the region of the outer layer part 12 under which the coil is not disposed. For example, in a case where the elongated medical instrument 1 is a balloon catheter, the region which is proximal to the balloon (the region in which the balloon is not formed) is an example of the substantially flat surface 121. In a case where the elongated medical instrument 1 is a guide wire, the region which is proximal to the area under which the coil on the distal end of the guide wire is formed (the region in which the coil is not provided) is an example of the substantially flat surface 121.

Those cases where the recesses 13 according to the present disclosure are irregularly formed on a surface other than the substantially flat surface 121 are also included in the scope of the present disclosure. For example, an elongated medical instrument in which the surface of the outer layer part 12 that corresponds to the coil shape is formed with recesses 13 in addition to the recesses and protrusions due to the coil shape is a type of the elongated medical instrument according to the present disclosure.

The shape of the recesses 13 may be that of an irregularly shaped linear portion. The shape of the recesses 13 may include a spatial frequency of 3 to 10 (l/mm).

The irregularly disposed (formed) and irregularly shaped recesses 13 can be expressed as irregularly shaped linear portions 13 that are irregularly formed on the surface of the outer layer part 12. Alternatively, the irregularly shaped recesses 13 can be described as irregularly shaped linear portions 13 originating from the cracks generated in the outer layer part 12.

Therefore, the elongated medical instrument according to the present embodiment may be expressed as including, for example, the base material part 11, and the outer layer part 12 made of a resin and provided on the surface of the base material part 11, in which a predetermined region 121 on the surface side of the outer layer part 12 is provided with a plurality of irregularly shaped linear portions 13 that are irregularly formed, and are linear portions 12 that are indented toward the base material part 11.

The elongated medical instrument according to the present embodiment may be expressed as including, for example, the base material part 11, and the outer layer part 12 made of a resin and provided on the surface of the base material part 11, in which a predetermined region 121 on the surface side of the outer layer part 12 is provided with a plurality of irregularly shaped linear portions 13 that are irregularly formed and originating from the cracks generated in the outer layer part 12, and are linear portions 12 that are indented toward the base material part 11.

The present embodiment will be described with the recesses being formed in a direction in which the outer diameter becomes smaller than that of the substantially flat surface 121 of the outer layer part 12, but an interpretation is possible in which, for example, the irregularly shaped protrusions are formed in a direction in which the outer diameter becomes larger than that of the diameter of the base material part 11.

Hereinafter, the guide wire 1 will be described as an example of an elongated medical instrument. However, the present disclosure can be applied not only to the guide wire 1 but also to the catheter 2. The irregularly shaped recesses can be irregularly formed on the outer layer part provided on the surface of the catheter 2. The present disclosure is applicable to not only a guide wire or the catheter 2, but also to elongated medical members for treatment (such as endoscopes) that are inserted through tubular living tissue such as a blood vessel or the gastrointestinal tract.

### [Example 1]

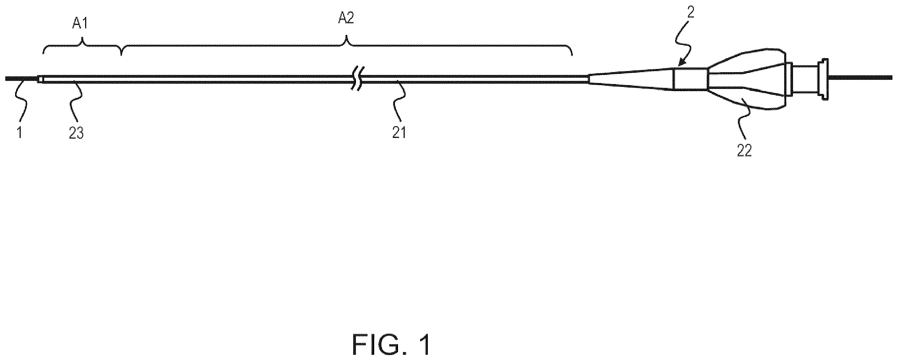

Example 1 will be described using FIG. 1 to FIG. 18. As shown in FIG. 1, a catheter 2 is used, for example, to diagnose or treat a constricted part or an occluded part. The catheter 2 may also be a balloon catheter, a microcatheter, a cardiac catheter, a pulmonary artery catheter, an angiographic catheter, a urethral catheter, a gastrointestinal catheter, or the like.

The catheter 2 includes a catheter shaft 21, and a connector 22 joined to the proximal end side of the catheter shaft 21. The catheter shaft 21, for example, is provided with a coil body, and a resin tube that covers the outside of the coil body (not illustrated). A lumen (not illustrated) through which the guide wire 1 or another catheter can be inserted is formed on the inner peripheral side of the coil body along the longitudinal direction of the coil body.

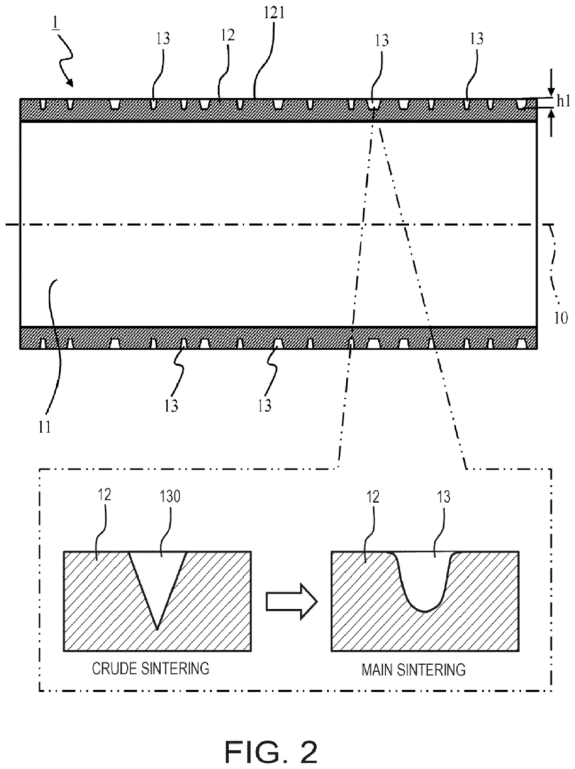

FIG. 2A is a longitudinal sectional view showing enlarged the guide wire 1 according to the present example. The guide wire 1 includes a core material 11, which is an example of a "base material part", an outer layer part 12 provided in close contact so as to cover the surface of the core material 11, and irregularly shaped recesses 13 that are irregularly formed on the surface of the outer layer part 12. The guide wire 1 may include a different resin layer or the like (not illustrated) between the core material 11 and the outer layer part 12. Note that a coil-shaped portion is sometimes provided on the distal end side of the core material 11, but this is not illustrated. FIG. 2B shows enlarged a region of the guide wire 1 in which the coil-shaped portion is not formed. The depth dimension h1 of the recesses 13 is not a constant value, and is variable. However, the depth dimension h1 of the recesses 13 does not exceed the thickness dimension of the outer layer part 12.

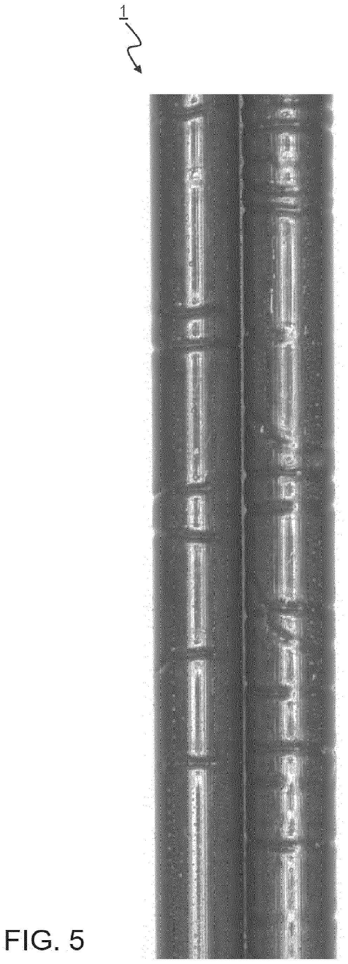

The irregularly shaped recesses 13 are, as shown in FIG. 5, a plurality of wrinkle shaped linear portions formed on the surface of the guide wire 1, and are recessed toward the core material 11 side.

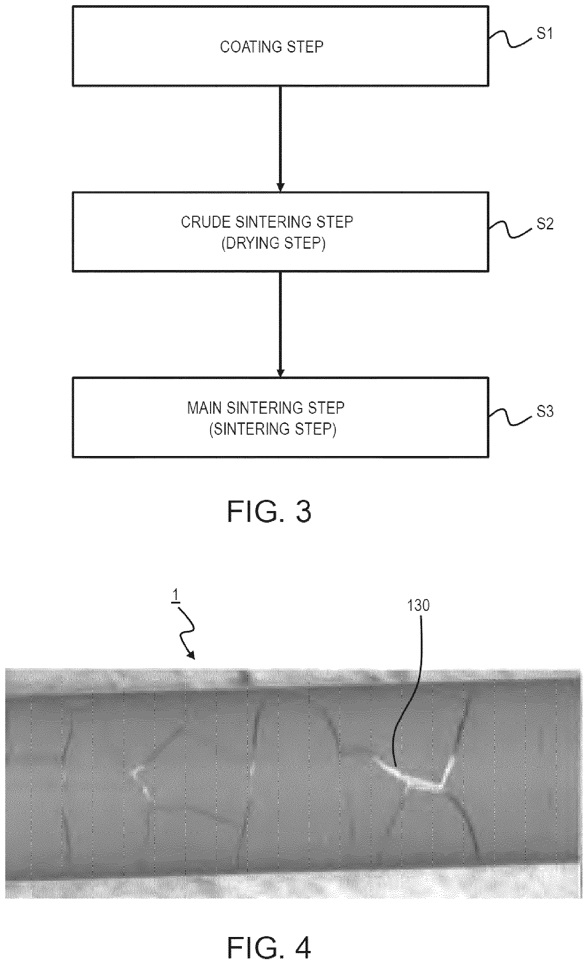

The lower part of FIG. 2 shows a schematic of a transverse cross-section of a recess 13. FIG. 3 shows a method of forming the recesses 13. The description will be continued referring to FIG. 2 and FIG. 3.

In the coating step S1, the core material 11 is dip-coated using a PFA dispersion, which is the material that forms the outer layer part 12. A PFA dispersion is a suspension in which PFA particles (dispersoid) are dispersed in a dispersion medium such as water. The entire circumference may be dip-coated over the entire length of the core material 11, or the entire circumference of a predetermined region of the core material 11 may be dip-coated. The dispersoid is not limited to PFA. Any thermoplastic resin having low frictional resistance can be used as the material of the outer layer part 12.

In the crude sintering step S2, the dip-coated core material 11 is dried by heating at a first temperature TEMP1 for a first time t1 to evaporate the dispersion medium. The crude sintering step can also be called a drying step.

The first temperature TEMP1 may be selected, for example, from a range of temperatures that are at least the evaporation temperature of the dispersion medium, and lower than the melting point of the dispersoid (evaporation temperature of dispersion medium ≤ TEMP1 < melting point of dispersoid). The first time t1 can be set to a predetermined time required for evaporation of the dispersion medium.

As shown in the lower left of FIG. 2, the crude sintering step S2 results in cracks 130 being generated in the outer layer part 12, which is made of a resin and provided on the surface of the guide wire 1. The lower left of FIG. 2 schematically shows a single crack 130 enlarged, but in reality, as shown in the external view in FIG. 4, a plurality of cracks 130 are formed on the surface of the guide wire 1 after the crude sintering step S2 is completed.

Returning to FIG. 3, in the main sintering step S3, the main sintering is performed by heating the guide wire 1, in which cracks 130 have been generated on the surface, to a second temperature TEMP2 for a second time t2. When the crude sintering step is referred to as the drying step, the main sintering step can also be referred to as the sintering step. The crude sintering step and the main sintering step may be performed consecutively in terms of time.

The second temperature TEMP2 is set higher than the first temperature TEMP1 used during crude sintering (TEMP1 < TEMP2). The second temperature TEMP2 is set to at least the melting point of the dispersoid (here, the melting point of PFA).

The second time t2 can be set to a time that allows the resin above the cracks 130 to melt and flow into the cracks 130, but prevents the cracks 130 from becoming completely buried with the resin that has flowed in. Next, the relationship between the first time t1 and the second time t2 will be described. The first time t1, which is the duration of the crude sintering, may be set to at least the second time t2, which is the duration of the main sintering (t1 ≥ t2). The first time t1 and the second time t2 may be set to the same value or similar values.

As shown in the lower left of FIG. 2, the recess 13 is formed as a result of the resin on the surface side of the outer layer part 12 melting and flowing into the crack 130. Because the recess 13 is formed due to the crack 130, it has the same irregular and linear shape as the crack 13. The transverse cross-section of the recess 13 is smoother than the transverse cross-section of the crack 130.

In this way, because the recesses 13 are formed due to the cracks 130 generated in the crude sintering step S2, they are irregularly shaped and linear recesses 13 that are irregularly generated on the surface of the outer layer part 12. A recess 13 that includes either or both of a curved portion and a straight portions can also be referred to as a wrinkle shape.

As mentioned above, FIG. 4 shows enlarged a portion of the surface of the guide wire 1 after completion of the crude sintering step S2. FIG. 5 shows enlarged a portion of the surface of the guide wire 1 after completion of the main sintering step S3. Because FIG. 4 and FIG. 5 show different locations enlarged, and also have different magnifications, the shapes of the cracks 130 and the shapes of the recesses 13 do not correspond, but in reality, as mentioned above, because the recesses 13 are formed as a result of the surrounding resin melting and flowing into the cracks 130, the shapes of the cracks 130 in plan view substantially correspond to the shapes of the recesses 13 in plan view. However, smaller cracks 130 may become filled due to the resin that has flowed in.

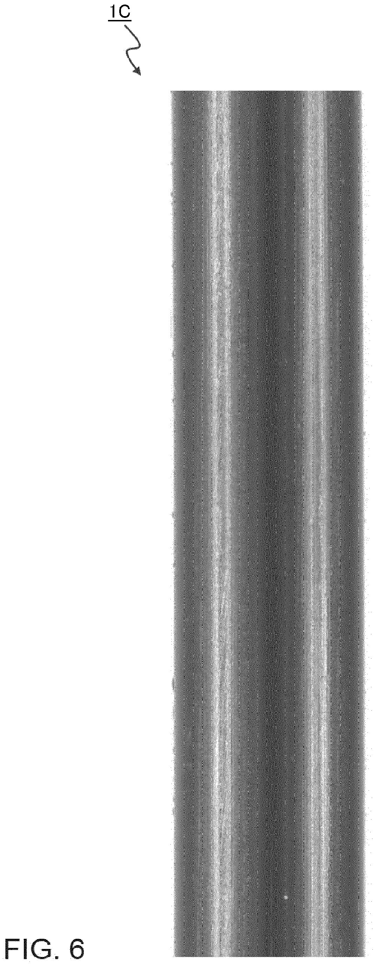

FIG. 6 shows enlarged a portion of a guide wire 1C according to a comparative example. The surface of the guide wire 1C according to the comparative example is not formed with irregularly shaped recesses and protrusions 13. If the guide wire 1C shown in FIG. 6 is observed at a higher magnification than the magnification of the external view in FIG. 5, recesses and protrusions would appear on the surface, though these are not intentionally formed.

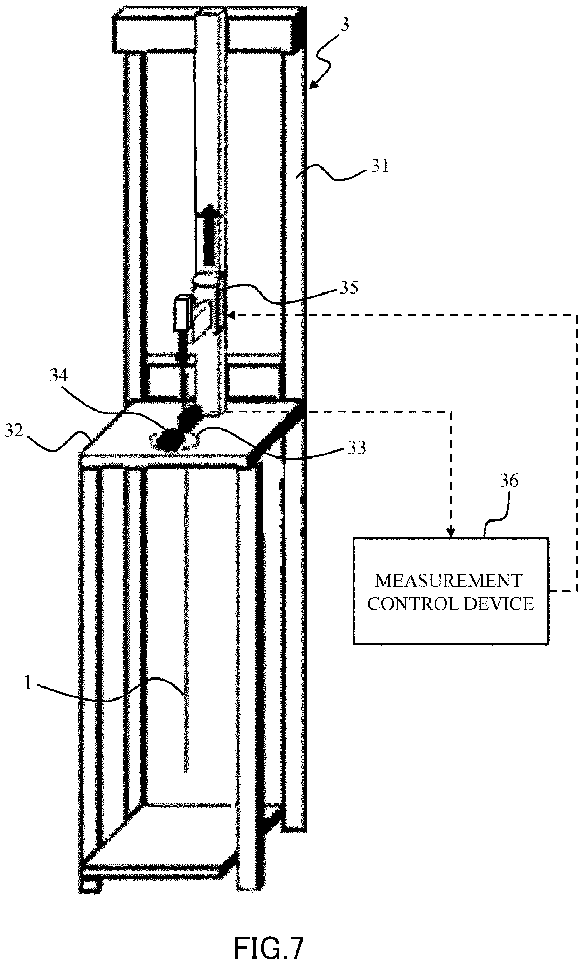

FIG. 7 shows a measurement device 3 that measures the outer diameter dimension of the guide wire 1. The outer diameter measurement device 3 includes, for example, a frame 31, a flat plate portion 32 having an insertion hole 33, an outer diameter measurement instrument 34, a linear actuator 35, and a measurement control device 36. As the outer diameter measurement instrument 34 and the measurement control device 36, it is possible to use, for example, a measurement head LS-9006 and a controller LS-9500 of the ultra-high speed and high precision dimension measurement instrument LS-9000 series from Keyence Corporation.

The frame 31 extends upward from a flat floor surface, and has the flat plate portion 32 attached midway along the vertical direction. The flat plate portion 32 is formed with the insertion hole 33, through which the guide wire 1 is inserted. The outer diameter measurement instrument 34 is disposed around the insertion hole 33. The outer diameter measurement instrument 34, for example, measures the outer diameter dimension of a target object using laser light, and a has a light emitter and a light receiver that are opposingly arranged so as to sandwich the insertion hole 33.

The linear actuator 35 is vertically provided on the flat plate portion 32 in a position slightly away from the insertion hole 33. The linear actuator 35 moves vertically upward at a constant speed according to a control signal from the measurement control device 36 while holding one end portion of the guide wire 1 (the upper end portion of the guide wire 1 in FIG. 7).

As the guide wire 1 is being pulled vertically at a constant speed by the linear actuator 35, the outer diameter measurement instrument 34 measures the outer diameter dimension of the guide wire 1 in real time, and outputs it to the measurement control device 36.

A measurement using the measurement device 3 in FIG. 7 can be performed, for example, using an outer diameter measurement sampling period of 8,000 times/sec, a vertical movement speed of 10 mm/sec, and a measurement length of 30 mm. The external dimension data of the guide wires of the present example and the comparative example obtained by such a measurement will be described below.

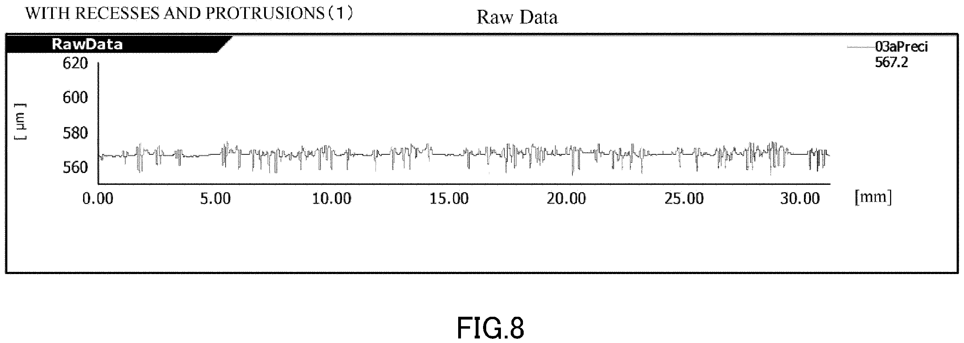

FIG. 8 is a graph showing a result (raw data) of measuring the outer diameter dimension of a guide wire 1 with irregularly shaped recesses and protrusions 13 that have been irregularly formed on the surface of the outer layer part 12 using the outer diameter measurement device 3 described in FIG. 7. The vertical axis represents the outer diameter dimension (pm), and the horizontal axis represents the position in the length direction of the guide wire 1. From the graph in FIG. 8, it can be seen that the surface of the guide wire 1 of the present example has recesses 13 that are irregularly formed with varying concavity.

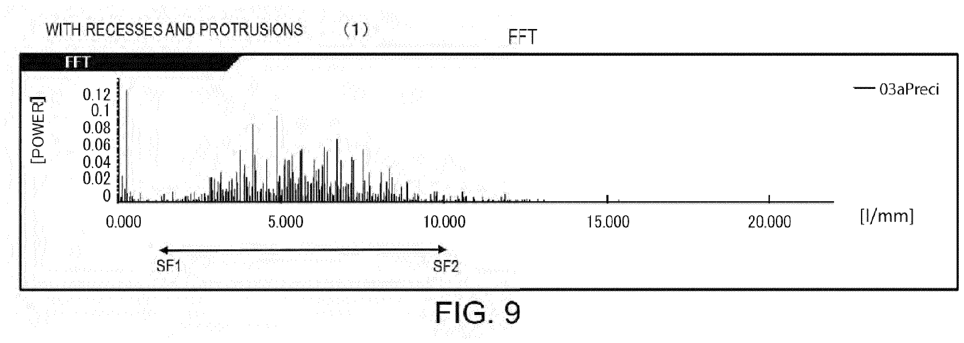

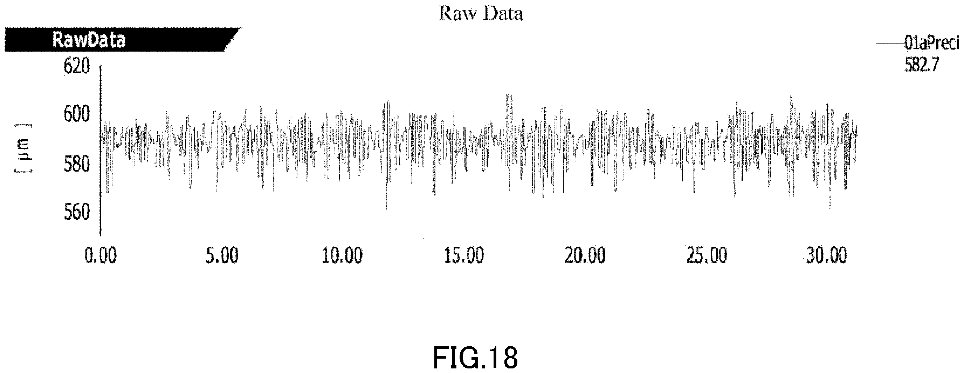

FIG. 9 is a waveform diagram showing the results obtained by an FFT analysis of the measurement data in FIG. 8. The vertical axis represents the power value, and the horizontal axis represents the spatial frequency (l/mm). According to the waveform diagram in FIG. 9, the waveform representing the recesses and protrusions 13 includes spatial frequencies in a range from a lower limit SF1 to an upper limit SF2. The lower limit SF1 is 1 (l/mm) and the upper limit SF2 is 10 (l/mm). That is, it can be determined from the experiment that the sliding resistance is reduced when recesses 13 including spatial frequencies of 1 to 10 (l/mm) are formed. Further, the effects described below in FIG. 18 can also be obtained when recesses and protrusions 13 having various shapes in plan view and in transverse cross-section are irregularly formed on the surface of the guide wire 1 so as to include spatial frequencies from 3 to 10 (l/mm).

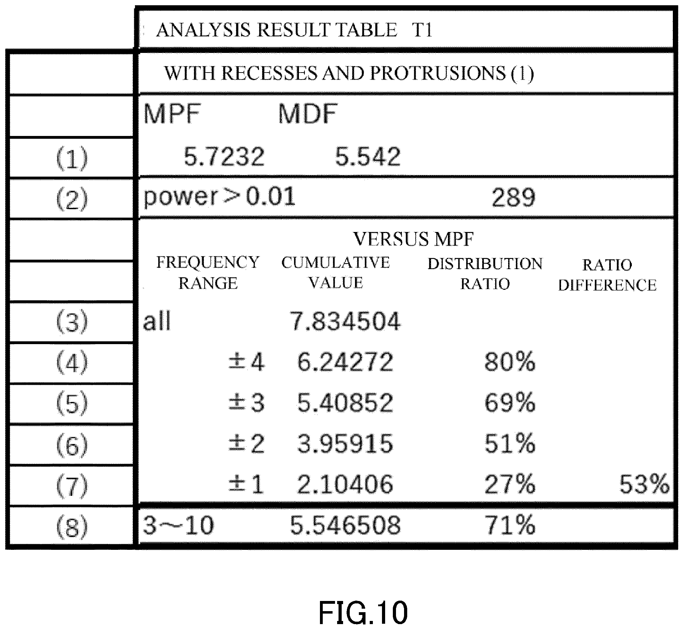

FIG. 10 is a table T1 showing FFT analysis results of the outer diameter dimension of a guide wire 1 having recesses 13.

As displayed in the first row (1) of table T1, in the guide wire 1 of the present example, the MPF is 5.7232 and the MDF is 5.542. As displayed in the second row (2) of table T1, there are 289 data points in which the power value is larger than 0.01.

The third row (3) to the seventh row (7) of table T1 represent the data distribution state when the spatial frequency range is changed with respect to the MPF. As shown in the third row (3), the cumulative power value in the entire spatial frequency range is 7.834504. As shown in the fourth row (4), the cumulative value in the spatial frequency range of MPF plus or minus 4 is 6.24272. The distribution ratio thereof is 80%. As shown in the fifth row (5), the cumulative value in the spatial frequency range of MPF plus or minus 3 is 5.40852. The distribution ratio thereof is 69%. As shown in the sixth row (6), the cumulative value in the spatial frequency range of MPF plus or minus 2 is 3.95915. The distribution ratio thereof is 51%. As shown in the seventh row (7), the cumulative value in the spatial frequency range of MPF plus or minus 1 is 2.10406. The distribution ratio thereof is 27%, and the ratio difference between the distribution ratio for a spatial frequency of plus or minus 4 and the distribution ratio for a spatial frequency of plus or minus 1 is 53%.

As shown in the eighth row (8) of table T1, the cumulative value is 5.546508 in the spatial frequency range of 3 to 10 (l/mm). The distribution ratio thereof is 71%.

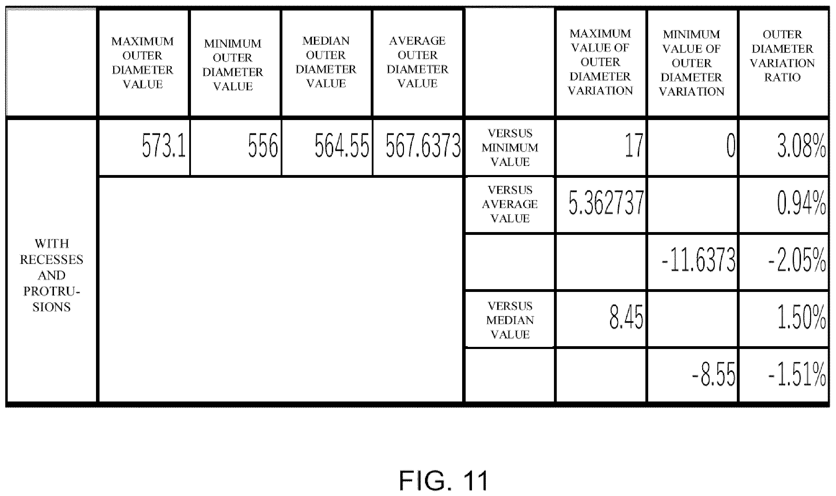

FIG. 11 is a table showing the results of measuring the outer diameter dimension of a guide wire 1 having recesses 13 in the outer layer part 12. The unit is pm. The maximum value of the outer diameter dimension is 573.1, the minimum value of the outer diameter dimension is 556, the median value of the outer diameter dimension is 564.55, the average value of the outer diameter dimension is 567.6373, and the variation value of the outer diameter dimension is 2.452621.

Focusing on the variation of the outer diameter dimension, the maximum value of the outer diameter variation with respect to the minimum value of the outer diameter dimension is 17, the minimum value of the outer diameter variation is 0, and the outer diameter variation ratio is 3.0%. The outer diameter variation value with respect to the average value of the outer diameter dimension is from 5.362737 to -11.6373, and the outer diameter variation ratio is from +0.94% to -2.05%. The outer diameter variation value with respect to the median value of the outer diameter dimension is from 8.45 to -8.55, and the outer diameter variation ratio is from +1.50% to -1.51%.

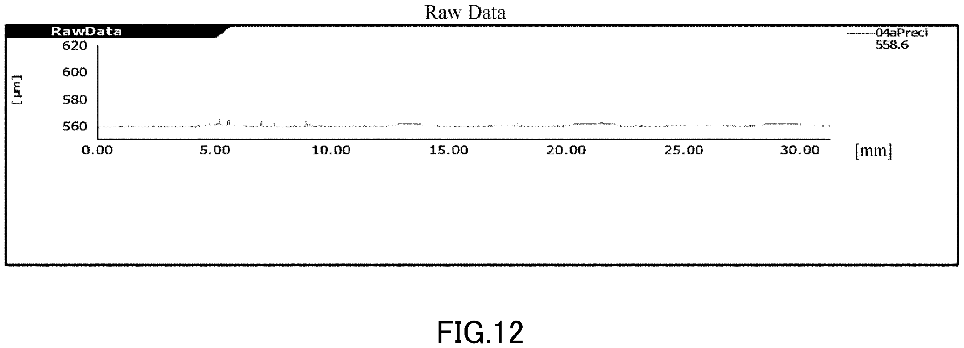

FIG. 12 is a graph of data obtained by measuring the outer diameter dimension of a guide wire 1C according to a comparative example along the axial direction. The vertical axis represents the outer diameter (pm), and the horizontal axis represents the position in the length direction of the guide wire 1. From the graph in FIG. 12, it can be seen that the surface of the guide wire 1C of the comparative example is smooth and without recesses and protrusions when compared to the guide wire shown in FIG. 8.

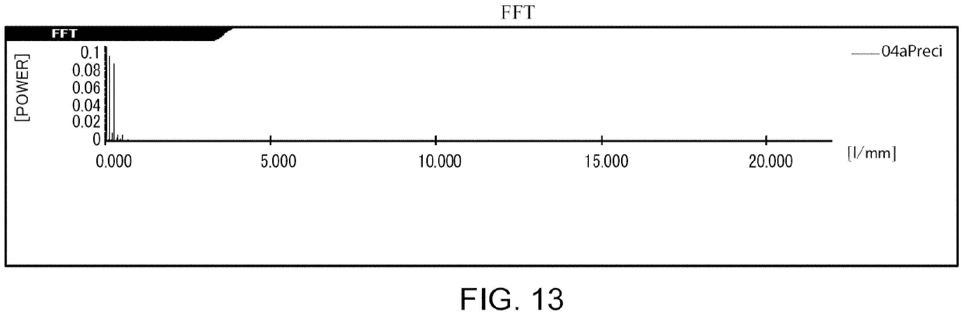

FIG. 13 is a waveform diagram showing the results obtained by an FFT analysis of the measurement data in FIG. 12. The vertical axis represents the power value, and the horizontal axis represents the spatial frequency (l/mm). According to the waveform diagram in FIG. 13, it can be seen that substantially no recesses and protrusions exist on the surface of the guide wire 1C according to the comparative example.

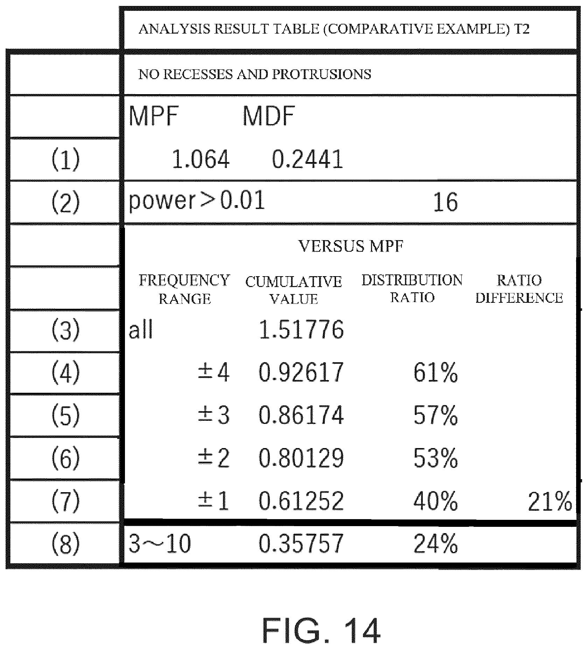

FIG. 14 is a table T2 showing FFT analysis results of the outer diameter dimension of the guide wire 1C of the comparative example.

As displayed in the first row (1) of table T1, in the guide wire 1C of the comparative example, the MPF is 1.064 and the MDF is 0.2441. As displayed in the second row (2) of table T2, there are no data points in which the power value is larger than 0.01. The divergence between the MPF value and the MDF value is due to the fact that the power value fluctuates at a level that does not appear in the graph at 1 (l/mm) or more in FIG. 13. Such fluctuations are caused by diameter fluctuations and the like that occur due to the processing of the core material 11 of the guide wire.

The third row (3) to the seventh row (7) of table T2 represent the data distribution state when the spatial frequency range is changed with respect to the MPF. As shown in the third row (3), the cumulative value in the entire spatial frequency range is 1.51776. As shown in the fourth row (4), the cumulative value in the spatial frequency range of MPF plus or minus 4 is 0.926171. The distribution ratio thereof is 61%. As shown in the fifth row (5), the cumulative value in the spatial frequency range of MPF plus or minus 3 is 0.861739. The distribution ratio thereof is 57%. As shown in the sixth row (6), the cumulative value in the spatial frequency range of MPF plus or minus 2 is 0.801288. The distribution ratio thereof is 53%. As shown in the seventh row (7), the cumulative value in the spatial frequency range of MPF plus or minus 1 is 0.612517. The distribution ratio thereof is 40%.

As shown in the eighth row (8) of table T2, the cumulative value is 0.357573 in the spatial frequency range of 3 to 10 (l/mm). The distribution ratio thereof is 24%.

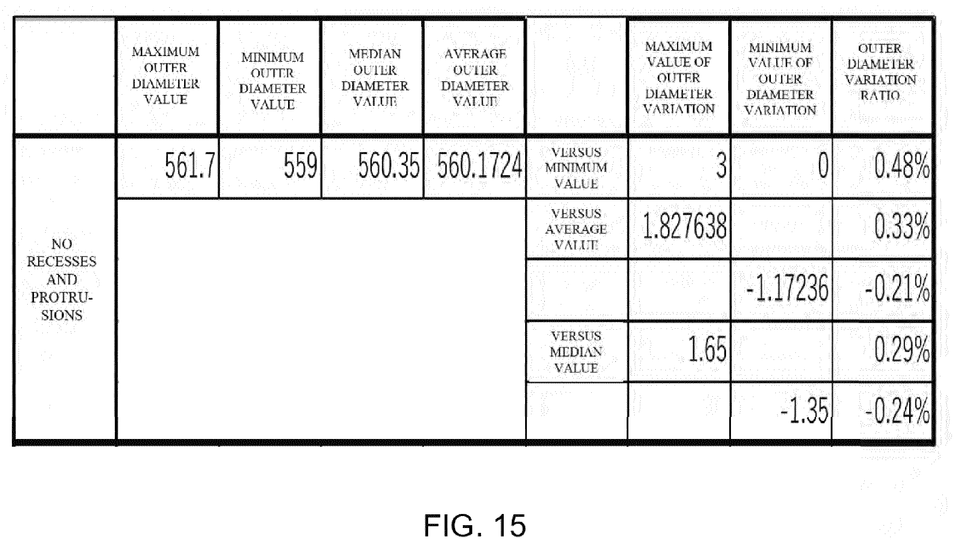

FIG. 15 is a table showing the results of measuring the outer diameter dimension of the guide wire 1C. The unit is pm. The maximum value of the outer diameter dimension is 561.7, the minimum value of the outer diameter dimension is 559, the median value of the outer diameter dimension is 560.35, the average value of the outer diameter dimension is 560.1724, and the variation value of the outer diameter dimension is 0.662483. Although the description is omitted, the surface of the guide wire 1C of the comparative example does not have the recesses 13 of the present example, and has almost no variation in the outer diameter because the surface is substantially smooth.

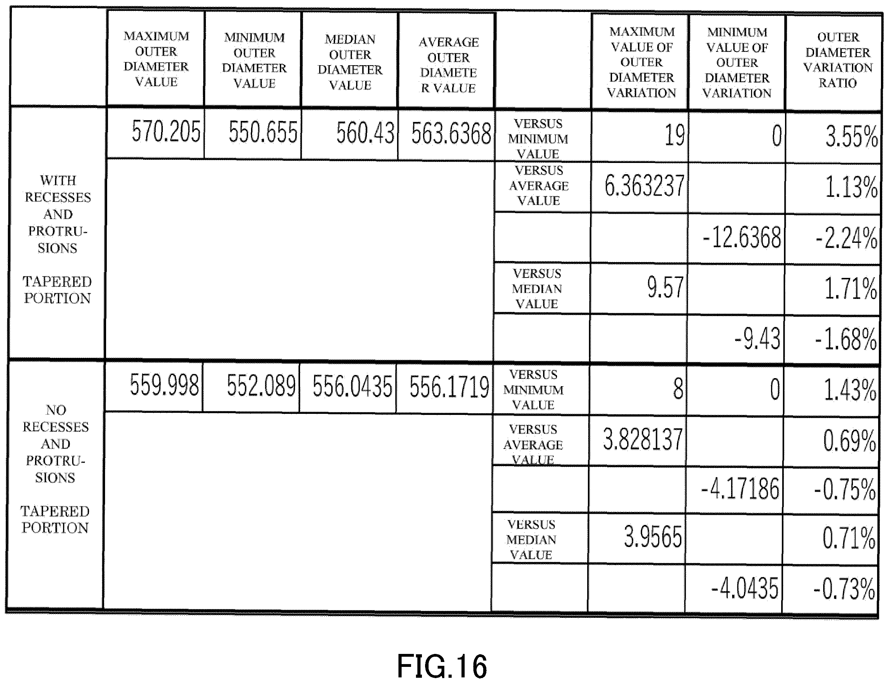

A case where the guide wire 1 has a tapered portion will be described using FIG. 16. FIG. 16 is a table showing the results of measuring the outer diameter dimension of the tapered portion of the guide wire 1.

Although the description is omitted, the guide wire 1 may have a tapered portion in which the diameter gradually decreases toward the distal end side. In the tapered portion of the guide wire 1, because a taper is formed in the core material 11, and recesses 13 are further formed on the surface of the guide wire 1, the change in the outer dimension of the tapered portion is greater than the change in the outer dimension in the non-tapered portion. Therefore, in order to determine the change in the outer dimension due to the formation of the recesses 13 in the tapered portion, it is necessary to obtain a numerical value of the change in the outer dimension that is corrected so as to offset the change in the diameter of the core material 11.

The correction of the change in the outer dimension is performed by approximating the change in the outer diameter of the core material 11 between the distal end and the proximal end of the tapered portion with a linear function, and using the inverse function of the linear function to add or subtract to the outer diameter value according to the distance from the distal end or the proximal end of the tapered portion.

The upper part of FIG. 16 shows analytical values of the measurement data for a case where recesses 13 are irregularly formed on the tapered portion when the core material 11 of the guide wire 1 has a diameter reduction of 8 pm per 10 cm in the longitudinal direction. The lower part of FIG. 16 shows analytical values of the measurement data for a case where recesses 13 are not formed on the tapered portion when the core material 11 of the guide wire 1C has a diameter reduction of 8 pm per 10 cm in the longitudinal direction. The unit is pm.

The upper part of the table will be described. The maximum value of the outer diameter dimension is 570.205, the minimum value of the outer diameter dimension is 550.655, the median value of the outer diameter dimension is 560.43, the average value of the outer diameter dimension is 563.6368, and the variation value of the outer diameter dimension is 3.269153.

Focusing on the variation of the outer diameter dimension, the maximum value of the outer diameter variation with respect to the minimum value of the outer diameter dimension is 19, the minimum value of the outer diameter variation is 0, and the outer diameter variation ratio is 3.55%. The outer diameter variation value with respect to the average value of the outer diameter dimension is from +6.363237 to -12.6368, and the outer diameter variation ratio is from +1.13% to -2.24%. The outer diameter variation value with respect to the median value of the outer diameter dimension is from +9.57 to -9.43, and the outer diameter variation ratio is from +1.71% to -1.68%.

Therefore, compared to a case where a taper is not present, because the variation in the outer dimension of the tapered portion increases, a correction of the outer dimension of the tapered portion is necessary.

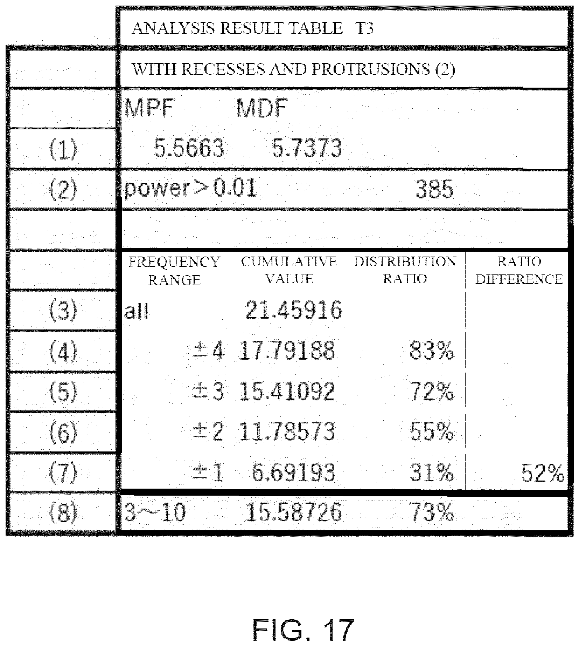

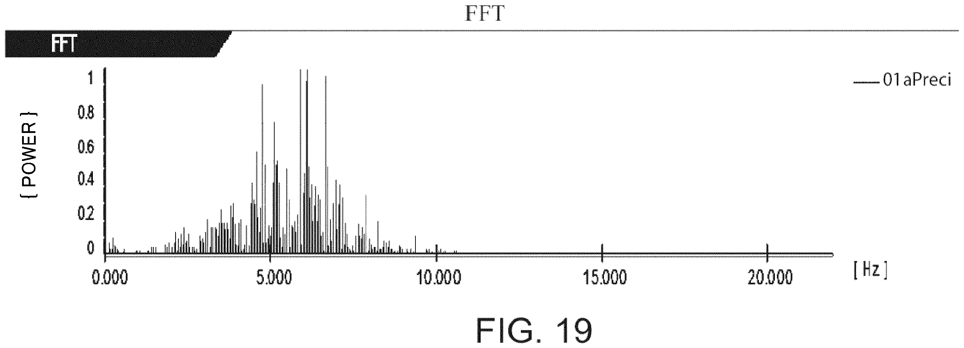

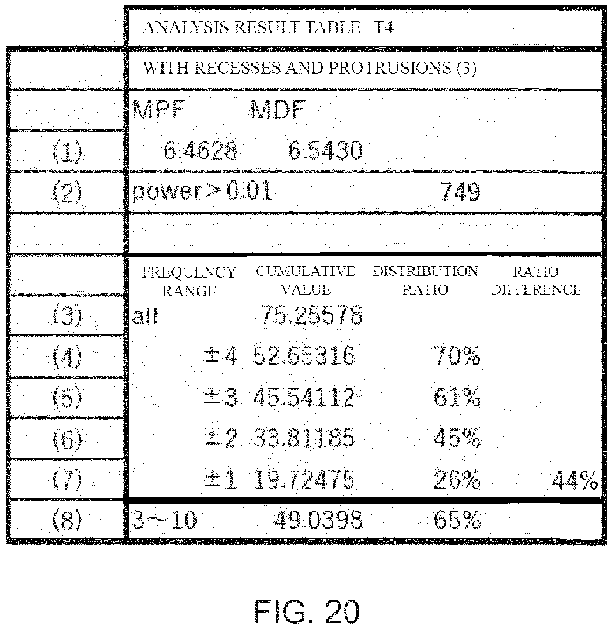

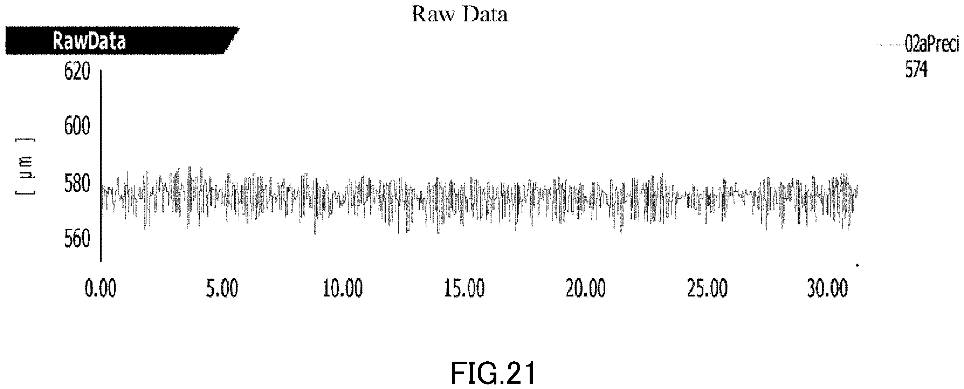

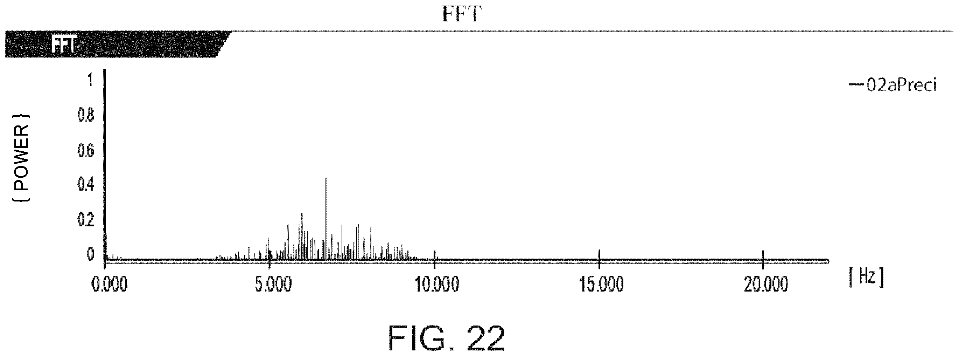

FIG. 17 to FIG. 22 will be described. FIG. 17 is an analysis result table T3 for another case where irregular recesses 13 are formed on the surface of the guide wire 1. FIG. 18 is a graph showing a measurement of an external dimension measurement. FIG. 19 is a graph showing FFT analysis results of the external dimension measurement data. FIG. 20 is an analysis result table T4 for yet another case where irregular recesses 13 are formed on the surface of the guide wire 1. FIG. 21 is a graph showing a measurement of an external dimension measurement. FIG. 22 is a graph showing FFT analysis results of the external dimension measurement data. The analysis result table T3 and the analysis result table T4 are examples where the thickness of the outer layer part 12 made of a resin is formed thicker than in the analysis result table T1 in FIG. 8 to FIG. 10.

As displayed in the first row (1) in tables T1, T3 and T4, the guide wire 1 formed with the recesses 13 has the MPF in the range of 4 to 7, and the MDF also in the range of 4 to 7. As displayed in the second row (2), there are at least 100 data points in which the power value is larger than 0.01.

As displayed in the third row (3) to seventh row (7) of tables T1, T3 and T4, the lower limit of the distribution ratio in a spatial frequency of plus or minus 4 is 66%. The upper limit of the distribution ratio in a spatial frequency of plus or minus 1 is 35%. The lower limit of the distribution ratio in a spatial frequency of plus or minus 4 is the median value of the minimum value of the fourth row (4) of tables T1, T3 and T4 in which recesses and protrusions are formed (70% in T4), and the value (61%) of the fourth row (4) of table T2 in which recesses and protrusions are not formed. The upper limit of the distribution ratio in a spatial frequency of plus or minus 1 is the median value of the maximum value of the seventh row (7) of tables T1, T3 and T4 in which recesses and protrusions are formed (31% in T3), and the value (40%) of the seventh row (7) of table T2 in which recesses and protrusions are not formed. The lower limit of the ratio difference in between the lower limit of the spatial frequency of plus or minus 4 and the upper limit of the spatial frequency of plus or minus 1 in the tables in which recesses and protrusions are formed is 31%.

As shown in the eighth row (8) of tables T1, T3 and T4, it can be seen that in the spatial frequency range of 3 to 10 (l/mm), the irregular recesses 13 according to the present example are formed on the surface of the catheter 1.

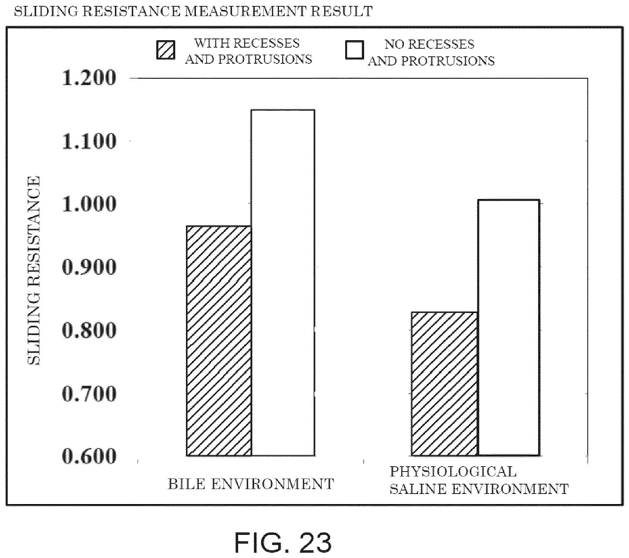

FIG. 23 is a graph showing a comparison of the sliding resistance of the guide wire 1 of the present example, which has the recesses 13 on the surface, and the guide wire 1C of the comparative example, which does not have recesses on the surface. The vertical axis represents the sliding resistance value. In FIG. 23, a simulated environment was used in which an endoscope was inserted from the oral cavity, through the esophagus and stomach to the duodenum, and a guide wire was inserted through the endoscope. The guide wire 1 and the guide wire 1C will be compared in a case that simulates introduction of the guide wire into an environment in which, after an endoscope is first inserted into the body in the simulated environment, the inside of the lumen of the endoscope through which the guide wire is inserted is filled with physiological saline, and a case that simulates introduction of the guide wire into an environment in which, after inserting the guide wire into an endoscope, bile flows backward from the distal end of the endoscope and causes the inside of the lumen through which the guide wire is inserted to be filled with bile. The shaded bar graph represents the guide wire 1 of the present example, and the white bar graph represents the guide wire 1C of the comparative example. In both simulation experiments, it can be seen that the guide wire 1 of the present example having irregular recesses 13 on the surface has a smaller sliding resistance.

According to the present example configured in this manner, the sliding resistance can be reduced by irregularly disposing the recesses 13 on the substantially flat surface 121 of the outer layer part 13. In particular, the shape of the recesses can reduce the sliding resistance by including a spatial frequency of 3 to 10 (l/mm).

It is clear from the first row (1) of tables T1 and T3 that the sliding resistance is reduced when the value of at least one of the MDF and MPF is 4 to 7 (l/mm). Similarly, it is clear from the first row (1) of tables T1 and T3 that the sliding resistance is reduced when the value of at least one of the MDF and MPF is 5 (l/mm) or more.

It is clear from the second row (2) of tables T1 and T3 that the sliding resistance is reduced when there are ten or more spatial frequencies with a power value of 0.01 or more.

It is clear from the FFT waveform diagram of FIG. 9 that the sliding resistance is reduced when the spatial frequencies with a power value of 0.01 or more are dispersed over a range of 1 to 10 (l/mm).

It is clear from the seventh row (7) of tables T1 and T3 that the sliding resistance is reduced when the distribution ratio in a range of the average frequency component value plus or minus 1 (l/mm) is less than 35%.

It is clear from the fourth row (4) of tables T1 and T3 that the sliding resistance is reduced when the distribution ratio in a range of the average frequency component value plus or minus 4 (l/mm) is about 80% or more.

It is clear from the ratio difference in the seventh row (7) of tables T1 and T3 that the sliding resistance is reduced when the difference between the distribution ratio of the average frequency component value plus or minus 1 (l/mm) and the distribution ratio of plus or minus 4 (l/mm) is about 50% or more.

It is clear from the eighth row (8) of table T1 that the sliding resistance is reduced when the power spectrum of spatial frequencies 3 to 10 (l/mm) is 50% or more. Similarly, it is clear from the eighth row (8) of table T1 that the sliding resistance is reduced when the power spectrum of spatial frequencies 3 to 10 (l/mm) is 70% or more.

It is clear from the outer diameter variation ratio in FIG. 11 that the sliding resistance is reduced when the outer diameter variation ratio is 3% or more with respect to the minimum outer diameter value.

It is clear from the graphs in FIG. 8, FIG. 18, and FIG. 22 that the sliding resistance is reduced when, in a range of 1 cm in the longitudinal axis direction, there are ten or more locations in which change in the outer diameter is 5 pm or more in a width of less than 1 mm.

It is clear from the measurement results when the recesses 13 are formed on the tapered portion shown in the upper part of FIG. 16 that the sliding resistance is reduced when, in a case where the outer layer part 12 includes a tapered portion, the outer diameter variation ratio excluding the change in outer diameter due to the tapered portion is 3% or more with respect to the minimum outer diameter value.

Although the disclosed embodiments have been described above, the present disclosure is not limited to the aforementioned embodiments and can be variously modified.

The embodiments described above have been described in detail to enable the disclosed embodiments to be understood more easily, and are not necessarily limited to those having all the described configurations. Furthermore, the configuration of an embodiment may be replaced with the configuration of another embodiment. Moreover, the configuration of another embodiment can be added to the configuration of an embodiment. In addition, portions of the configuration of each embodiment can be added, deleted, or replaced with other configurations.

Furthermore, the technical features included in the embodiments described above are not limited to the combinations explicitly stated in the claims, and can be combined as appropriate.

### DESCRIPTION OF REFERENCE NUMERALS

1 Guide wire
1C Guide wire according to comparative example
2 Catheter
11 Base material part
12 Outer layer part
13 Recess
130 Crack

## Claims

1. An elongated medical instrument comprising:
a base material part; and
an outer layer part made of a resin and provided on a surface of the base material part,
wherein a substantially flat surface of the outer layer part has irregularly disposed recesses or protrusions.

2. The elongated medical instrument according to claim 1, wherein the substantially flat surface is a surface corresponding to a region of the outer layer part under which a coil is not disposed.

3. The elongated medical instrument according to claim 1, wherein a shape of the recesses is an irregularly-shaped linear portion.

4. The elongated medical instrument according to claim 3, wherein a waveform shape exhibited by an outer diameter having the recesses or the protrusions includes a spatial frequency of 3 to 10 (l/mm).

5. An elongated medical instrument comprising:
a base material part; and
an outer layer part made of a resin and provided on a surface of the base material part,
wherein:
a substantially flat surface of the outer layer part has irregularly disposed recesses or protrusions, and
when a measurement result of an outer diameter of the outer layer part is analyzed by FET (Fast Fourier Transform) along a longitudinal axis direction of the outer layer part, at least one of Conditions 1 to 9 below is satisfied:
(Condition 1) a value of at least one of a median power frequency (MDF) or a mean power frequency (MPF) is 4 to 7 (l/mm);
(Condition 2) at least one of the MDF or the MPF is 5 (l/mm) or more;
(Condition 3) there are 100 or more spatial frequencies in which the power value is 0.01 or more;
(Condition 4) the spatial frequencies with a power value of 0.01 or more are distributed from 1 to 10 (l/mm);
(Condition 5) a distribution ratio in a range of an average frequency component value plus or minus 1 (l/mm) is less than 35%;
(Condition 6) a distribution ratio in a range of the average frequency component value plus or minus 4 (l/mm) is approximately 70% or more;
(Condition 7) a difference between the distribution ratio in the range of the average frequency component value plus or minus 1 (l/mm) and the distribution ratio of plus or minus 4 (l/mm) is approximately 40% or more;
(Condition 8) a power spectrum of the spatial frequencies 3 to 10 (l/mm) is 50% or more; and
(Condition 9) a power spectrum of the spatial frequencies 3 to 10 (l/mm) is 65% or more.

6. An elongated medical instrument comprising:
a base material part; and
an outer layer part made of a resin and provided on a surface of the base material part;
wherein:
a substantially flat surface of the outer layer part has irregularly disposed recesses or protrusions, and
a measurement result of an outer diameter of the outer layer part along a longitudinal axis direction of the outer layer part satisfies at least one of Conditions 10 and 11 below:
(Condition 10) an outer diameter variation ratio with respect to a minimum outer diameter value is 3% or more; and
(Condition 11) in a range of 1 cm in the longitudinal axis direction, there are ten or more locations in which a change in the outer diameter is 5 µm or more in a width of less than 1 mm.

7. The elongated medical instrument according to claim 6, wherein:
in Condition 10, in a case where the outer layer part includes a tapered portion, the outer diameter variation ratio excluding a change in the outer diameter due to the tapered portion is 3% or more with respect to a minimum outer diameter value.

8. The elongated medical instrument according to any one of claims 1 to 7, wherein the outer layer part comprises any one of PFA (perfluoroalkoxyalkane), FEP (tetrafluoroethylene-hexafluoropropylene copolymer), or ETFE (tetrafluoroethylene-ethylene copolymer).

9. The elongated medical instrument according to any one of claims 1 to 7, wherein the elongated medical instrument is a guide wire.

10. The elongated medical instrument according to claim 9, wherein an outer diameter dimension of the guide wire is 1 mm or less.

11. The elongated medical instrument according to claim 9, wherein an outer diameter dimension of the guide wire is 700 pm or less.

12. The elongated medical instrument according to claim 9, wherein an average value of an outer diameter dimension of the guide wire is 500 to 600 pm.

13. The elongated medical instrument according to claim 1, wherein the elongated medical instrument is a catheter.
